# NEW EUROPEAN PATENT SPECIFICATION

(11) **EP 0 920 300 B2**
(45) Date of publication and mention of the opposition decision: **10.01.2007**
(45) Mention of the grant of the patent: 09.04.2003
(21) Application number: 97924162.7
(22) Date of filing: 03.06.1997
(51) Int. Cl.: A61K 8/92, A61Q 17/04, A61Q 19/00, A23L 1/30

(54) **VEGETABLE OIL COMPOSITIONS**
PFLANZLICHE ÖLZUSAMMENSETZUNGEN
COMPOSITIONS D'HUILE VEGETALE

(30) Priority: 03.06.1996 GB 9611529; 03.06.1996 GB 9611530; 03.06.1996 GB 9611531
(43) Date of publication of application: 09.06.1999
(73) Proprietor: CRODA INTERNATIONAL plc, Goole North Humberside DN14 9AA (GB)
(72) Inventor: COUPLAND, Keith, Hotham, York YO4 3UX (GB); NICHOLS, Julie, Ann, Hibaldstow, N. Lincolnshire DN20 9ES (GB)
(74) Representative: Coates, Ian Harold
(86) International application number: PCT/GB1997/001497
(87) International publication number: WO 1997/046220

(56) References cited:
- EP-A- 0 421 867
- EP-A- 0 430 870
- EP-A- 0 454 102
- EP-A- 0 457 950
- EP-A- 0 460 848
- EP-A- 0 711 503
- FR-A- 2 648 347
- FR-A- 2 679 109
- GB-A- 2 118 567
- US-A- 3 405 151
- US-A- 5 223 285
- BARRE ET AL.: "The effect of Borage Oil Consumption on the Composition of Individual Phospholipids in Human Platelets", LIPIDS, , , vol. 27, no. 5, pages 315 to 320
- JOURNAL OF THE AMERICAN OIL CHEMITS'SOCIETY , C.R.SMITH ET AL ,41,1964 , P-290-291
- JOURNAL OF THE AMERICAN OIL CHEMISTS'SOCIETY ,R,KLEINMAN,41,1964,P 459-460

## Description

This invention relates to the use of oils containing glycerides of essential fatty acids, or of essential fatty acids formed from such oils, for topical and dietetic purposes. The invention also relates to compositions including such oils or essential fatty acids.

Fats and oils are triesters of glycerol; they are known as triglycerides or triacylglycerols. If the triglyceride is solid at room temperature, then it is generally considered to be a fat, whereas if it is liquid at room temperature, then it is generally considered to be an oil. Most triglycerides in animals are fats, while most triglycerides in vegetables tend to be oils. Fatty acids can be obtained from fats or oils by hydrolysis.

Certain fatty acids, called essential fatty adds, must be present in the human diet and are used in the body to synthesise, for example, prostaglandins. There are two main series of essential fatty acids: one is called the n3 family (also known as the ω-3 family); and the other is called the n6 family (also known as the ω-6 family).

Stearidonic acid (SA) is a polyunsaturated fatty acid of the n3 family. Chemically it can be described as 6c9c12c15c-octadecatetraenoic acid or 18:4n3. Other essential fatty adds are linoleic acid (LA), α-linolenic add (ALA) and γ-linolenic add (GLA) - these adds can be described as C18:2n6, C18:3n3 and C18:3n6 respectively.

It is known that the oils of the pips of certain fruits contain triglycerides of a number of fatty adds. In GB-A-2118567 it is disclosed that an oil-extracted from the pips of the fruit of blackcurrants, redcurrants and gooseberries contain triglycerides of fatty adds in the proportions shown in Table 1.

**Table 1**

| Fatty Acid | Blackcurrants | Red Currents | Gooseberries |
|---|---|---|---|
| C16:0 | 6-7 wt% | 4-5 wt% | 7-8 wt% |
| C18:0 | 1-2 wt% | 1-2 wt% | 1-2 wt% |
| C18:1 cis | 9-10 wt% | 14-15 wt% | 15-16 wt% |
| C18:1 trans | 0.5 wt% | 0.5-1 wt% | 1-2 wt% |
| C18:2n6 | 47-49 wt% | 41-42 wt% | 29-41 wt% |
| C18:3n6 | 15-19 wt% | 4-5 wt% | 10-12 wt% |
| C18:3n3 | 12-14 wt% | 29-31 wt% | 19-20 wt% |
| C18:4n3 | 3-4 wt% | 2.5-3.5 wt% | 4-5 wt% |

Stearidonic acid is found in marine oils, some plant oils (EP-A-0454102, US-A-5223285, FR-A-2679109, US-A-3405151) and in lipids isolated from micro-organisms. It is produced in the human body by the biochemical transformation of linolenic acid. The metabolic transformation in the human body of n3 fatty adds by desaturation and elongation takes place as follows:

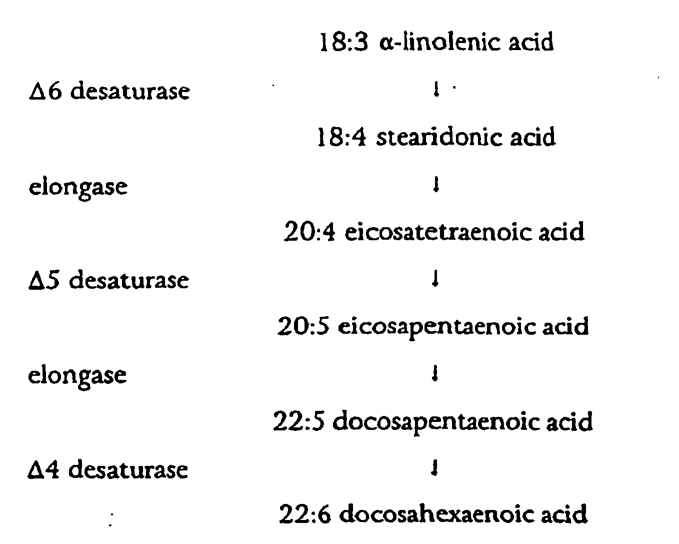

The activity of the enzyme "Δ6 desaturase" is known to be of lower activity following certain illnesses, and in old age; its activity can also be lowered by poor diet and certain lifestyles. This is significant, because if the activity of Δ6 desaturase is lowered, then the body's capacity to make stearidonic acid (and the other compounds in the scheme shown above) is also lowered. One way to remedy this problem is to take a dietary supplement containing stearidonic acid.

None of the materials in Table 1 is very useful as a source of stearidonic acid, because this acid is present in such a low concentration. In order to use these materials as a source of stearidonic acid, it would be necessary eitherto use them in large quantities or to carry out expensive chemical processing to concentrate the stearidonic acid. Accordingly, there is a need for a material that is rich in stearidonic acid but that does not contain any toxic compounds.

We have found a rich natural, non-toxic, source of stearidonic acid that can be used to make a wide range of dietetic, cosmetic and healthcare products. In particular, we have found that the oil of seeds of the *Boraginaceae family* are a rich source of stearidonic acid. We have also found that the oil itself can be used in dietetic, cosmetic and healthcare products, without the need for additional treatment or purification.

In one aspect, the invention relates to the use of an oil extracted from seeds of the *Boraginaceae* family in topical application to, or oral ingestion by, the human or animal body. This oil alone may be used for these purposes or, preferably, it is used to form a part of a composition for topical application to, or oral ingestion by, the human or animal body. The invention therefore provides a composition for use as a source of stearidonic acid to supplement that produced by the human or animal body, which composition comprises in the range of from 1 to 20% by weight of the total composition of an oil extracted from the seeds of the genus *Echium,* which oil comprises in the range of from 5 to at least 20% stearidonic acid triglycerides, by weight of the total fatty acids in the oil extract, which stearidonic acid triglycerides may optionally have been converted to a physiologically acceptable derivative thereof, and optionally a physiologically acceptable carrier therefor.

In an especially advantageous embodiment, the seeds are of the genus *Echium;* in particular, we have found that the seeds of *Echium vulgaris* and *Echium plantagineum* are very useful.

The oil extracted from the seeds of the *Boraginaceae* family contains the triglyceride of stearidonic acid. In general, the oil contains stearidonic acid (in the form of the triglyceride) in an amount greater than 5wt%, preferably greater than 5.5 wt%, more preferably greater than 10 wt%, and most preferably greater than 15 wt%; the oil may contain as much as 20 wt% stearidonic acid, or more. We can obtain the oil at these concentrations without carrying out any purification process to increase the concentration of stearidonic acid in the oil. We have analysed the composition of three oils formed from the seeds of the Borignaceae family, and found that the fatty acids were present (in the form of triglycerides) in the amounts shown in Table 2.

**Table 2**

| Fatty Acid | E.vulgaris | E. Plantagineum | TrichodesmaZeylanicum |
|---|---|---|---|
| 16:0 | 6.2 wt% | 7.6 wt% | 9.4 wt% |
| 18:0 | 2.0 wt% | 3.8 wt% | 5.8 wt% |
| 18:1 | 8.0 wt% | 16.7 wt% | 26.8 wt% |
| 18:2 (LA) | 10.3 wt% | 16 wt% | 18.2 wt% |
| 18:3 (GLA) | 5.3 wt% | 11.9 wt% | 5.5 wt% |
| 18:3 (ALA) | 47.3 wt% | 29.9 wt% | 26.8 wt% |
| 18:4 (SA) | 19.8 wt% | 12.3 wt% | 5.7 wt% |
| Other | 1.1 wt% | 1.8 wt% | 1.8 wt% |

The oil extract can be used in a wide variety of topical applications, including many cosmetic and dermatological applications. For example, the oil extract can be used in skin creams and emulsions, including cleansers, moisturising creams, and sun screens; shampoos; and bath oils. The oil also has a wide range of dietetic uses. For example, the oil can be provided as an additive to existing food products, for instance as an additive to milk or milk-based drinks; it may form be in the form of a dietary supplement, or part of a dietary supplement (eg a vitamin containing supplement), and may be provided in solid form, for example as a tablet or as a soft or hard gelatin capsule, or in liquid form.

The oil extract itself, or compositions formed from the oil extract, can be used to treat a wide variety of skin disorders, such as dry skin, itchy skin, psoriasis, eczema and the like.

According to another aspect of the invention there is provided a composition for topical application to, or oral ingestion by, the human or animal body, comprising 1-20% w/w of an oil extracted from the seeds of the genus *Echium,* as defined above, in combination with a physiologically acceptable carrier.

The precise nature of the carrier depends on the use desired for the composition. In addition, the carrier would usually contain other active ingredients, such as moisturiser (*eg* for moisturising cream), a surfactant (egfor shampoo) or a UV-blocking/absorbing compound (*eg* for a sun cream). Many specific examples of suitable carriers are disclosed in the Examples below.

The amount of oil in the composition also depends upon the desired use. However, an amount of the oil from 1wt% and 20wt% is appropriate.

We have described above the use of oil extracted from the seeds of the *Boraginaceaefamily,* genus *Echium,* and compositions including such oils. This oil contains glycerides of a number of fatty acids, including stearidonic acid. However, it is possible to process the oil extracted from the seeds to convert it at least partially to the corresponding fatty acids of the triglycerides in the oil. This processing would typically comprise a saponification to step to hydrolyse the triglycerides, an acidification step, and at least one separation step to recover the stearidonic acid and other fatty acids. There may also be a purification step to purify the stearidonic acid.

The present invention relates to the use of stearidonic acid formed from an oil extracted from seeds of the genus *Echium* in topical application to, or oral ingestion by, the human or animal body. The stearidonic acid so formed is preferably used to form part of a composition for topical application, to or oral ingestion by, the human or animal body. The stearidonic acid may be part of a mixture of essential fatty acids formed from an oil extracted from the seeds of the genus *Echium.*

The following examples illustrate the invention.

### Example 1

10 kg of the seeds of *Echium plantagineum* were crushed and the oil was extracted with 15 litres of petroleum ether (BP 40-60°C). The petroleum ether extract was evaporated to yield 1741g of a golden yellow oil. The oil was converted to the corresponding fatty acid methyl esters and was analysed by gas chromatography. The lipid profile was as follows:

| Fatty Acid | Fatty Acid Content |
|---|---|
| 16:0 | 7.2% |
| 18:0 | 4.0% |
| 18:1 | 18.2% |
| 18:2 (LA) | 16.5% |
| 18:3(GLA) | 11.8% |
| 18:3(ALA) | 28.9% |
| 18:4(SA) | 12.2% |
| Other | 1.2% |

The values obtained in this example are slightly different from those in Table 2 due to insignificant experimental errors.

### Example 2

20g of the seeds of *Trichodesma zeylanicum* seeds were crushed and placed in a Soxhlet extraction thimble. An oil was isolated by refluxing n-hexane around the crushed seed to produce a solution of the oil in n-hexane. The oil was separated from the n-hexane by roto-evaporation; 5g of a green oil was recovered.

An aliquot of the oil was converted to the corresponding fatty acid methyl esters and was analysed by gas chromatography. The lipid profile was as follows:

| Fatty Acid | Fatty Acid Content |
|---|---|
| 16:0 | 9.4 wt% |
| 18:0 | 5.7 wt% |
| 18:1 | 25.5 wt% |
| 18:2 (LA) | 19.0 wt% |
| 18:3 (GLA) | 5.5 wt% |
| 18:3 (ALA) | 26.5 wt% |
| 18:4 (SA) | 5.9 wt% |
| Other | 2.5 wt% |

The values in obtained in this example are slightly different from those in Table 2 due to insignificant experimental errors.

### Example 3

A sun screen oil was prepared from the following ingredients:

| | |
|---|---|
| Butyl methoxydibenzoylmethane (Parsol 1789)(1) | 2.0 wt% |
| Octyl methoxycinnamate (Parsol MCX)(1) | 7.5 wt% |
| Benzophenone-3 (Uvinul M40)(2) | 4.5 wt% |
| PPG-2 myristyl ether proprionate(Promyristyl PM3)(3) | 10.0 wt% |
| Oil from Example 1 | 2.0-10.0 wt% |
| Caprylic/capric triglycerides (Crodamol GTCC) | to 100 wt% |
| Perfume, preservatives, colour | qs |

| | |
|---|---|
| (1) From Givaudan (2) From BASF (3) From Croda | |

The sun screen oil was formed by blending the ingredients, heating gently. The resultant blend was stirred to cool.

### Example 4

A physical block sun screen cream was prepared from the following ingredients:

| Oil Phase: | |
|---|---|
| Myristyl myristate (Crodamol MM)(1) | 1.5 wt% |
| Dimethicone (silicone 200/200cs)(2) | 7.0 wt% |
| Glyceryl stearate (GMS N/E)(1) | 2.5 wt% |
| Stearic acid (Crosterene)(1) | 3.0 wt% |
| PVP/eicosene copolymer (Antaron V220)(3) | 0.5 wt% |
| Nonionic emulsifying blend (Polawax GP200)(1) | 2.0 wt% |
| Octyl hydroxystearate (Crodamol OHS)(1) | 5.0 wt% |
| Oil from Example 1 | 2.0-10.0 wt% |
| Super refined shea butter(1) | 5.0 wt% |
| Caprylic/capric triglycerides (Crodamol GTCC)(1) | 9.0 wt% |
| Titanium dioxide | 5.0 wt% |

| Water Phase: | |
|---|---|
| Deionised water | to 100 wt% |
| Flobeads (CL-2080)(5) | 5.0 wt% |
| Glycerine | 3.0 wt% |
| Triethanolamine | 0.9 wt% |
| Perfume, preservatives, colour | qs |

| | |
|---|---|
| (1) From Croda (2) From Dow Corning (3) From GAF (4) From K&K Greef (5) From Landsdowne Chemicals | |

The sun screen cream was made by heating the oil phase (including titanium dioxide) and water phase separately to 80-85°C, then adding the water phase to the oil phase with vigorous agitation. The triethanolamine was then added. At 45°C the Flobeads were added. The stirring was continued until the temperature reached about 55°C, and then the composition was passed through suitable homogenising equipment, such as a triple roll mill).

### Example 5

A shampoo was prepared from the following ingredients:

| | |
|---|---|
| Sodium laureth sulphate (Empicol ESB3)(1) | 50.0 wt% |
| Cocamidopropyl betaine (Incronam 30)(2) | 5.0 wt% |
| Oil from Example 1 | 1-5 wt% |
| Polysorbate-20 (Crillet 1)(2) | 3.0 wt% |
| PPG-5-ceteth- 10-phosphate (Crodafos SG)(2) | 3.0 wt% |
| Triethanolamine | to pH 6.5-7.0 |
| Butylated hydroxytoluene | 0.01 wt% |
| Deionised water | to 100 wt% |
| Perfume, preservatives, colour | qs |

| | |
|---|---|
| (1) From Albright & Wilson (2) From Croda | |

The shampoo was prepared by first forming a premix by solubilising the essential fatty acid containing oil from Example 1 in the surfactant mixture (polysorbate-20 and PPG-5-ceteth-10-phosphate). The other ingredients were combined and, when homogeneous, they were added to the premix. The resultant mixture was stirred until clear.

### Example 6

A cleanser was prepared from the following ingredients:

| Oil Phase: | |
|---|---|
| Nonionic emulsifying wax (Polawax GP200)(1) | 2.0 wt% |
| Cetearyl alcohol (Crodacol CS90EP)(1) | 1.0 wt% |
| Glyceryl stearate (Cithrol GMS N/E DP2186)(1) | 1.0 wt% |
| Caprylic/capric triglycerides (Crodamol GTCC)(1) | 8.0 wt% |
| Rosehip oil (2) | 1.0 wt% |
| Oil from Example 1 | 1-10 wt% |

| Water Phase: | |
|---|---|
| Glycerin | 2.0 wt% |
| Carbomer 981 (carbopol 981 - 2% aqueous solution)(3) | 5.0 wt% |
| Triethanolamine | to pH 6.5 |
| Deionised water | to 100 wt% |
| Perfume, preservative, colour | qs |

| | |
|---|---|
| (1) From Croda (2) From Novarom (3) From B F Goodrich | |

The cleanser was prepared by heating the water and oil phase separately to 65-70°C, then adding the water phase to the oil phase with stirring. The pH was then adjusted to pH 6.5 with the triethanolamine. The resultant mixture was stirred to cool.

### Example 7

A moisturising facial oil was prepared from the following ingredients:

| | |
|---|---|
| Oil from Example 1 | 5.0-15.0 wt% |
| Tocopheryl acetate(2) | 5.0 wt% |
| butylated hydroxytoluene | 0.05 wt% |
| Caprylic/capric triglycerides (Crodamol GTCC)(1) | to 100 wt% |
| Perfume, preservatives, colour | qs |

| | |
|---|---|
| (1) From Croda (2) From Roche | |

The facial oil was prepared by incorporating the butylated hydroxytoluene in the caprylic/capric triglyceride with gentle warming and stirring. The resultant mixture was stirred to cool, then the remaining ingredients were added, whilst stirring.

### Example 8

A moisturising body cocktail was prepared from the following ingredients:

| | |
|---|---|
| Oil from Example 1 | 5.0 to 20.0 wt% |
| Super refined grapeseed oil(1) | 20.0 wt% |
| Tocopheryl acetate(2) | 2.0 wt% |
| Caprylic/capric triglycerides (Crodamol GTCC)(1) | to 100 wt% |
| Perfume, preservatives, colour | qs |

| | |
|---|---|
| (1) From Croda (2) From Roche | |

The body cocktail was prepared by combining the preservatives and the caprylic/capric triglycerides with gentle warming and stirring. The resultant mixture was stirred to cool. The remaining ingredients were added, whilst stirring.

### Example 9

A dispersible bath oil was made from the following ingredients:

| | |
|---|---|
| Oil from Example 1 | 1.0 to 5.0 wt% |
| Tocopheryl acetate (2) | 3.0-5.0 wt% |
| Laureth-3 (Volpo L3 Special)(1) | 12.5 wt% |
| Isopropyl myristate (Crodamol IPM)(1) | 20.0 wt% |
| Caprylic/capric triglycerides (Crodamol GTCC)(1) | to 100 wt% |
| Perfume, preservatives, colour | qs |

| | |
|---|---|
| (1) From Croda (2) From Roche | |

The bath oil was prepared by adding the preservatives to the caprylic/capric triglycerides with gentle warming and stirring. The remaining ingredients were added, and the resultant mixture was stirred to cool.

### Example 10

A skin lotion was prepared from the following ingredients:

| Oil Phase: | |
|---|---|
| Oil from Example 2 | 5.0-10.0 wt% |
| C₁₀-C₃₀ cholesterol/lanosterol esters (Super Sterol ester)(1) | 1.0 wt% |
| Stearic acid (Crosterene SA4130)(1) | 2.0 wt% |
| Glyceryl stearate S/E (GMS S/E GE0802)(1) | 2.0 wt% |
| Nonionic emulsifying wax (Polawax GP200)(1) | 0.75 wt% |

| Water Phase: | |
|---|---|
| Glycerin | 6.0 wt% |
| Triethanolamine | 0.9 wt% |
| Deionised water | to 100 wt% |
| Perfume, preservatives, colour | qs |

| | |
|---|---|
| (1) From Croda | |

The skin lotion was prepared by heating the oil and water phases separately to 65-70°C, then adding the water phase to the oil phase while stirring. The resultant mixture was stirred to cool, and the perfume was added at 45°C.

### Example 11

An emollient skin cream was prepared from the following ingredients:

| Oil Phase: | |
|---|---|
| Oil from Example 2 | 10 wt% |
| Nonionic emulsifying wax (Polawax GP200)(1) | 4.0 wt% |
| Synthetic beeswax (Syncrowax BB4)(1) | 2.0 wt% |
| Myristyl myristate (Crodamol MM)(1) | 1.0 wt% |
| Butylated hydroxytoluene | 0.05 wt% |

| Water Phase: | |
|---|---|
| Carbomer 941 (Carbopol 941)(2) | 0.2 wt% |
| Triethanolamine | 0.2 wt% |
| Deionised water | to 100 wt% |
| Perfume, preservatives colour | qs |

| | |
|---|---|
| (1) From Croda (2) From B F Goodrich | |

The skin cream was prepared by hydrating the Carbopol 941 in water at 60-70°C, then adding the rest of the aqueous phase components at a similar temperature while stirring. The oil phase components were heated to 65-70 °C, then the water phase components were added to the oil phase components at 65°C whilst stirring. The perfume was added when the cream had cooled to below 45°C.

### Example 12

An emollient skin cream was prepared from the following ingredients:

| Oil Phase: | |
|---|---|
| Oil from Example 2 | 20 wt% |
| Nonionic emulsifying wax (Polawax GP200)(1) | 5.0 wt% |
| Synthetic beeswax (Syncrowax BB4)(1) | 2.0 wt% |
| Cetostearyl stearate (Crodamol CSS(1) | 5.0 wt% |
| Butylated hydroxytoluene | 0.05 wt% |

| Water Phase: | |
|---|---|
| Carbomer 941 (Carbopol 941)(2) | 0.2 wt% |
| Triethanolamine | 0.2 wt% |
| Glycerin | 3.0 wt% |
| Deionised water | to 100 wt% |
| Perfume, preservatives colour | qs |

| | |
|---|---|
| (1) From Croda (2) From B F Goodrich | |

The skin cream was prepared by hydrating the Carbopol 941 in water at 60-70°C, then adding the rest of the aqueous phase components at a similar temperature while stirring. The oil phase components were heated to 65-70°C, then the water phase components were added to the oil phase components at 65°C whilst stirring. The perfume was added when the cream had cooled to below 45°C.

### Example 13

An emollient dispersible bath oil was prepared from the following ingredients:

| | |
|---|---|
| PEG-20 Evening Primrose glycerides (Crovol EP40)(1) | 15.0 wt% |
| Oleyl alcohol (Novol)(1) | 17.5 wt% |
| Octyl palmitate (Crodamol OP)(1) | 8.0 wt% |
| Oil from Example 2 | 1.0 to 20.0 wt% |
| Light mineral oil (25cS/25°C) | to 100 wt% |
| Perfume, preservatives, colour | qs |

| | |
|---|---|
| (1) from Croda | |

The bath oil was prepared by blending all the ingredients together at ambient temperature.

### Example 14

A lipid emulsion for dietetic use was prepared from the following ingredients:

| | |
|---|---|
| Oil from Example 1 | 10.0 wt% |
| Lecithin (Centomix E)(1) | 2.0 wt% |
| Sorbitan monolaureate (Crill 1)(2) | 1.66 wt% |
| Polysorbate-20 (Crillet 1)(2) | 0.34 wt% |
| Distilled water | 86 wt% |

| | |
|---|---|
| (1) From Stern (2) From Croda | |

The emulsion was prepared by adding the emulsifiers to the oil, then adding the water while homogenising the mixture in a suitable mixer, such as a homogeniser. The mixture was homogenised for 5 minutes. The resultant emulsion can be used for human ingestion as a dietary supplement. It can be used as an additive for milk or milk drinks.

### Example 15

A lipid emulsion for dietetic use was prepared from the following ingredients:

| | |
|---|---|
| Oil from Example 2 | 10.0 wt% |
| Sorbitan monolaureate (Crill 1)(1) | 1.66 wt% |
| Polysorbate-20 (Crillet 1)(1) | 0.34 wt% |
| Distilled water | 88 wt% |

| | |
|---|---|
| (1) From Croda | |

The emulsion was prepared by adding the emulsifiers to the oil, then adding the water while homogenising the mixture in a suitable mixer, such as a homogeniser. The mixture was homogenised for 5 minutes. The resultant emulsion can be used for human ingestion as a dietary supplement. It can be used as an additive for milk or milk drinks.

Whilst certain embodiments of the invention have been described above, it will be appreciated that modifications can be made.

## Claims

1. A composition for use as a source of stearidonic acid to supplement that produced by the human or animal body, which composition comprises in the range of from 1 to 20% by weight of the total composition of an oil extracted from the seeds of *Echium* genus, which oil comprises in the range of from 5 to at least 20% stearidonic acid triglycerides, by weight of the total fatty acids in the oil extract, which stearidonic acid triglycerides may optionally have been converted to a physiologically acceptable derivative thereof, and optionally a physiologically acceptable carrier therefor.

2. A composition according to claim 1, wherein the oil is an extract from seeds of *Echium plantagineum.*

3. A composition according to claim 1 or claim 2 in a form suitable for dietetic use.

4. A composition according to any preceding claim, wherein the oil is added to food products.

5. A composition according to any preceding claim, wherein the oil is in the form of a dietary supplement.

6. A composition according to any preceding claim, in a form selected from the group consisting of milk, milk-based drinks, vitamin supplements, dietary supplements and lipid emulsions.

7. A composition according to claim 1 or claim 2 in a form suitable for cosmetic use.

8. A composition according to claim 1 or claim 2 in a form suitable for topical use.

9. A composition according to any of claims 1, 2 or 7 to 8, in a form selected from: skin creams, skin emulsions, cleansers, moisturising creams, sunscreens, sun creams, shampoos, facial oils, bath oils, body cocktails and skin lotions.

10. A composition according to any preceding claim, in solid form.

11. A composition according to any of claims 1 to 9, in liquid form.

12. A composition according to any preceding claim, wherein the oil comprises at least 10 wt% of the triglyceride of stearidonic acid, or a derivative thereof.

13. A composition according to any preceding claim, wherein the derivative is the corresponding fatty acid methyl ester.

14. A composition according to any preceding claim, wherein the oil comprises at least 12.2 wt% of the methyl ester of stearidonic acid.

15. A dietary composition comprising in the range of from 1 to 20% by weight of the total composition of an extract of an oil extracted from the seeds of Echium species, which oil comprises in the range of from 5 to at least 20% stearidonic acid triglyceride and 80 or less to 95% other fatty acid triglycerides selected from the triglycerides of 16:0, 18:0, 18:1, 18:2 (LA), 18:3 (GLA), 18:3 (ALA) and minor amounts of other fatty acid triglycerides, by weight of the total fatty acids in the oil extract, which stearidonic acid triglycerides or other fatty acid(s) may optionally have been converted to a physiologically acceptable derivative thereof, and optionally a physiologically acceptable carrier therefor, whereby the composition is capable of supplementing stearidonic acid produced by the human or animal body.

16. A composition according to claim 15, wherein the oil is an extract from *Echium plantagineum.*

17. The use of a composition according to any preceding claim in the preparation of a medicament for supplementing stearidonic acid in the human or animal body.

## Patentansprüche

1. Zusammensetzung zur Verwendung als Ausgangsstoff von Stearidonsäure, um diejenige zu ergänzen, die durch den menschlichen oder tierischen Körper erzeugt wird, welche Zusammensetzung in dem Bereich von 1 bis 20 Gew.-% der Gesamtzusammensetzung eines Öls, extrahiert aus den Samen der Gattung *Echium,* umfaßt, welches Öl in dem Bereich von 5 bis mindestens 20%, bezogen auf das Gewicht der Gesamtfettsäuren in dem Ölextrakt, Stearidonsäuretriglyceride umfaßt, welche Stearidonsäuretriglyceride gegebenenfalls in ein physiologisch verträgliches Derivat davon umgewandelt worden sein können, und gegebenenfalls ein physiologisch verträglicher Träger dafür.

2. Zusammensetzung nach Anspruch 1, wobei das Öl ein Extrakt aus den Samen von *Echium plantagineum* ist.

3. Zusammensetzung nach Anspruch 1 oder Anspruch 2 in einer Form, geeignet zum Nahrungsgebrauch.

4. Zusammensetzung nach einem vorhergehenden Anspruch, wobei das Öl zu Lebensmittelprodukten hinzugesetzt wird.

5. Zusammensetzung nach einem vorhergehenden Anspruch, wobei das Öl in der Form eines Nahrungsmittelzusatzes ist.

6. Zusammensetzung nach einem vorhergehenden Anspruch, in einer Form, ausgewählt aus der Gruppe, bestehend aus Milch, auf Milch basierenden Getränken, Vitaminzusätzen, Nahrungsmittelzusätzen und Lipidemulsionen.

7. Zusammensetzung nach Anspruch 1 oder Anspruch 2 in einer Form, geeignet zum kosmetischen Gebrauch.

8. Zusammensetzung nach Anspruch 1 oder Anspruch 2 in einer Form, geeignet zum topischen Gebrauch.

9. Zusammensetzung nach einem der Ansprüche 1, 2 oder 7 bis 8, in einer Form, ausgewählt aus: Hautcremes, Hautemulsionen, Reinigungsmitteln, Feuchtigkeitscremes, Sonnenschutzmitteln, Sonnencremes, Shampoos, Gesichtsölen, Badeölen, Körpercocktails und Hautlotionen.

10. Zusammensetzung nach einem vorhergehenden Anspruch in fester Form.

11. Zusammensetzung nach einem der Ansprüche 1 bis 9 in flüssiger Form.

12. Zusammensetzung nach einem vorhergehenden Anspruch, wobei das Öl mindestens 10 Gew.-% des Triglycerids von Stearidonsäure oder einem Derivat davon umfaßt.

13. Zusammensetzung nach einem vorhergehenden Anspruch, wobei das Derivat der entsprechende Fettsäuremethylester ist.

14. Zusammensetzung nach einem vorhergehenden Anspruch, wobei das Öl mindestens 12,2 Gew.-% des Methylesters von Stearidonsäure umfaßt.

15. Nahrungsmittelzusammensetzung, umfassend in dem Bereich von 1 bis 20 Gew.-% der Gesamtzusammensetzung einen Extrakt von einem Öl, extrahiert aus den Samen der Spezies Echium, welches Öl in dem Bereich von 5 bis mindestens 20% Stearidonsäuretriglycerid und 80 oder weniger bis 95% andere Fettsäuretriglyceride, ausgewählt aus den Triglyceriden von 16:0, 18:0, 18:1, 18:2 (LA), 18:3 (GLA), 18:3 (ALA), und kleinere Mengen anderer Fettsäuretriglyceride, bezogen auf das Gewicht der gesamten Fettsäuren in dem Ölextrakt, umfaßt, welche Stearidonsäuretriglyceride oder andere Fettsäure(n) gegebenenfalls in ein physiologisch verträgliches Derivat davon umgewandelt worden sein können, und gegebenenfalls einen physiologisch verträglichen Träger dafür, wobei die Zusammensetzung imstande ist, Stearidonsäure, erzeugt durch den menschlichen oder tierischen Körper, zu ergänzen.

16. Zusammensetzung nach Anspruch 15, wobei das Öl ein Extrakt aus *Echium plantagineum* ist.

17. Verwendung einer Zusammensetzung nach einem vorhergehenden Anspruch in der Zubereitung eines Medikaments zum Ergänzen von Stearidonsäure in dem menschlichen oder tierischen Körper.

## Revendications

1. Composition pour une utilisation en tant que source d'acide stéaridonique pour compléter celui qui est produit par un corps humain ou animal, laquelle composition comprend dans l'intervalle de 1 à 20% en poids de la composition totale d'une huile extraite de graines du genre *Echium,* laquelle huile comprend dans l'intervalle de 5 à au moins 20% de triglycérides d'acide stéaridonique, en poids de la totalité des acides gras dans l'extrait d'huile, lesquels triglycérides d'acide stéaridonique peuvent éventuellement avoir été convertis en un dérivé physiologiquement acceptable de ceux-ci, et éventuellement un véhicule physiologiquement acceptable pour celle-ci.

2. Composition suivant la revendication 1, dans laquelle l'huile est un extrait de graines de *Echium plantagineum.*

3. Composition suivant la revendication 1 ou la revendication 2, sous une forme appropriée pour un usage diététique.

4. Composition suivant l'une quelconque des revendications précédentes, dans laquelle l'huile est ajoutée à des produits alimentaires.

5. Composition suivant l'une quelconque des revendications précédentes, dans laquelle l'huile est sous la forme d'un complément diététique.

6. Composition suivant l'une quelconque des revendications précédentes, sous une forme choisie dans le groupe constitué de lait, de boissons lactées, de compléments vitaminés, de compléments diététiques et d'émulsions lipidiques.

7. Composition suivant la revendication 1 ou la revendication 2, sous une forme appropriée pour un usage cosmétique.

8. Composition suivant la revendication 1 ou la revendication 2, sous une forme appropriée pour un usage topique.

9. Composition suivant l'une quelconque des revendications 1, 2 ou 7 à 8, sous une forme choisie parmi: des crèmes pour la peau, des émulsions pour la peau, des démaquillants, des crèmes hydratantes, des écrans solaires, des crèmes solaires, des shampooings, des huiles pour le visage, des huiles pour le bain, des mélanges pour le corps et des lotions pour la peau.

10. Composition suivant l'une quelconque des revendications précédentes, sous une forme solide.

11. Composition suivant l'une quelconque des revendications 1 à 9, sous une forme liquide.

12. Composition suivant l'une quelconque des revendications précédentes, dans laquelle l'huile comprend au moins 10% en poids du triglycéride d'acide stéaridonique ou d'un dérivé de celui-ci.

13. Composition suivant l'une quelconque des revendications précédentes, dans laquelle le dérivé est l'ester de méthyle d'acide gras correspondant.

14. Composition suivant l'une quelconque des revendications précédentes, dans laquelle l'huile comprend au moins 12,2% en poids de l'ester de méthyle de l'acide stéaridonique.

15. Composition diététique comprenant dans l'intervalle de 1 à 20% en poids de la composition totale d'un extrait d'une huile extraite de graines du genre *Echium,* laquelle huile comprend dans l'intervalle de 5 à au moins 20% de triglycérides d'acide stéaridonique et de 80 ou moins à 95% de triglycérides d'autres acides gras choisis parmi les triglycérides de 16:0, 18:0, 18:1, 18:2 (LA), 18:3 (GLA), 18:3 (ALA) et des quantités mineures de triglycérides d'autres acides gras, en poids de la totalité des acides gras dans l'extrait d'huile, lesquels triglycérides d'acide stéaridonique ou d'un ou d'autres acides gras peuvent éventuellement avoir été convertis en un dérivé physiologiquement acceptable de ceux-ci, et éventuellement un véhicule physiologiquement acceptable pour celle-ci, en conséquence de quoi la composition est capable de compléter de l'acide stéaridonique produit par un corps humain ou animal.

16. Composition suivant la revendication 15, dans laquelle l'huile est un extrait de *Echium plantagineum.*

17. Utilisation d'une composition suivant l'une quelconque des revendications précédentes dans la préparation d'un médicament pour compléter de l'acide stéaridonique dans un corps humain ou animal.
